# EUROPEAN PATENT APPLICATION

(11) **EP 0 979 838 A1**
(43) Date of publication of application: **16.02.2000**
(21) Application number: 98115152.5
(22) Date of filing: 12.08.1998
(51) Int. Cl.: C08J 5/18, C08J 9/00, B29C 67/20, A61F 13/15, A61L 15/00, C08K 3/26, C08L 23/08

(54) **Breathable microporous film**

(71) Applicant: THE PROCTER & GAMBLE COMPANY, Cincinnati, Ohio 45202 (US)
(72) Inventor: Bonelli, Guido, 65125 Pescara (IT); Cimini, Carmine, 65126 Pescara (IT); Veglio, Paolo, 65126 Pescara (IT)
(74) Representative: Hirsch, Uwe Thomas M.H.

(57) **Abstract**

A breathable microporous film having improved extensibility is disclosed. The microporous breathable film is formed from a mixture of a thermoplastic polymer and of particles of an inorganic filler, and is provided with breathability by stretching it in at least one direction. The microporous film is provided with a better extensibility by selecting the particle size of the inorganic filler, and the moisture content of the particles of inorganic filler and of the thermoplastic polymer when they are blended together to provide the mixture, and by controlling the Dart Resistance of the film. A composite layered breathable structure comprising the microporous film is also disclosed, and moreover a disposable absorbent article comprising a microporous breathable film which is imparted extensibility by means of mechanical straining.

## Description

### Field of the Invention

The present invention relates to a breathable microporous film having improved extensibility. The present invention also relates to a breathable laminated composite structure comprising the breathable microporous film, which can be used as a breathable and liquid impermeable backsheet in a disposable absorbent article, and to a disposable absorbent article comprising the microporous breathable film having improved extensibility.

### Background of the Invention

A microporous film having breathability is well known and used for various consumer products such as packaging film and absorbent articles. There are prior art which are directed to improvement of such a microporous film, such as U.S. Patent 4,923,650 published on May 8, 1990, JP Patent publication 93/230252-A published on September 7, 1993, JP Patent publication 96/225680-A published on September 3, 1996, JP Patent publication 94/62794-B published on August 17, 1994, JP Patent publication 95/231913-A published on September 5, 1995, JP Patent publication 96/300436-A published on November 19, 1996, JP Patent publication 96/300498-A published on November 19, 1996, JP Patent publication 96/300499-A published on November 19, 1996, JP Patent publication 96/300500-A published on November 19, 1996, and JP Patent publication 87/167332-A published on July 23, 1987. The microporous film described in the prior art worked quite well as a backsheet of an absorbent article which requires breathability and liquid impermeability. There are also prior art which are directed to a process for making a microporous film and the microporous film made by the process, such as U.S. Patent 4,116,892 published on September 26, 1978, U.S. Patent 4,153,751 published on May 8, 1979, and U.S. Patent 4,289,831 published on September 15, 1981. These prior art disclose processes using a process of stretching a material to make a microporous film. However, none of the prior art discloses a microporous film having extensibility or a process to make a microporous film having extensibility so that a part of a microporous film is extensible. These prior art are directed to a technology to make non-microporous film microporous, but not a technology to make a microporous film extensible.

Absorbent articles such as a sanitary napkin having a portion of extensibility are disclosed in prior art, such as PCT publication WO 97/12576 published on April 10, 1997, PCT publication WO 96/12462 published on May 1, 1996, U.S. Patent 5,389.094 published on February 14, 1995 and U.S. Patent 5,704,930 published on January 6, 1998. In these disclosures, the flaps of the sanitary napkin are provided with extensibility for relieving the stresses that develop in the flaps when the flaps are folded down and under a wearer's undergarment. The extensibility can be provided by a number of different processes. For example, the extensibility on the flaps can be provided by mechanically straining, corrugating, "ring-rolling", heating and deforming, subjecting portions of the flaps to compression between mating plates, and the like. These processes include the process of applying a strain to a material to mechanically and permanently deform the material. Extensibility on the material is provided by remaining permanent deformation on the material. Therefore, a degree of extensibility is determined by a degree of an applied strain. The more extensibility is required, the higher strain is applied to the material.

As described above, microporous films are commonly used for a breathable backsheet of an absorbent article. Microporous films typically comprise a blend of a thermoplastic polymer and an inorganic filler such as calcium carbonate. The blend undergoes pore formation upon stretching as the inorganic filler separates from the polymer due to stress concentration. The formation of micropores permits the film to be breathable allowing the passage of vapor through the micropores while retarding the passage of liquid. While microporous films have good breathability, microporous films have lower "strain at break" than an ordinary non-microporous film. Therefore, if microporous films are subjected to high strain beyond the strain at break of the microporous film to obtain extensibility of the microporous film, such high strain causes many visible pin holes in the area where the strain is applied.

Based on the foregoing, there is a need for a microporous breathable film having an increased extensibility while having lesser visible pin holes. There is also a need for a disposable absorbent article comprising a microporous breathable film with improved extensibility, for example as part of a composite laminated structure of a breathable, liquid impermeable backsheet, wherein at least part of said film is made extensible by straining at least part of the microporous breathable film. None of the existing art provides all of the advantages and benefits of the present invention.

### Summary of the Invention

A breathable microporous film formed from a mixture of a thermoplastic polymer and particles of an inorganic filler, which is rendered microporous by stretching it at least in one direction. The particles of inorganic filler have a particle size of 10 µm or less, and the particles of inorganic filler and the thermoplastic polymer have a moisture content of less than 500 ppm when they are blended to provide the mixture. The breathable microporous film also has a Dart Resistance, as measured according to the Free Falling Dart Test referred to herein, of at least 160 g.

### BRIEF DESCRIPTION OF THE DRAWINGS

FIG. 1 is a top plan view of a sanitary napkin comprising the breathable microporous film of the present invention.
FIG. 2 is a lateral cross-sectional view taken along line 2-2 of Figure 1 through the corner region of the flaps of the sanitary napkin.
FIG. 3 is a fragmentary enlarged cross-sectional view of a part of the absorbent core and a part of the backsheet of the sanitary napkin.
FIG. 4 is a side elevational view of the ring rolling unit used for making the sanitary napkin.
FIG. 5 is a front elevational view of the ring rolling unit used for making the sanitary napkin.
FIG. 6 is a fragmentary enlarged cross sectional view of the tooth engagement of the ring rolling unit shown in FIG. 5.
FIG. 7 is a section view of a prior art breathable microporous film.

### Detailed Description of the Invention

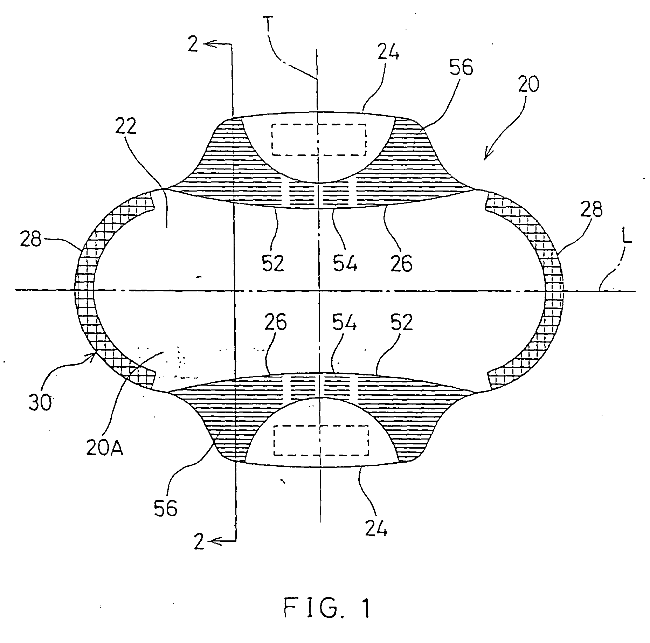

A preferred embodiment of a sanitary napkin 20 comprising the breathable microporous film of the present invention is shown in FIG. 1. As shown in FIG. 1, the sanitary napkin 20 basically comprises an absorbent means (or "main body portion") 22, and two flaps 24. The sanitary napkin 20 has two surfaces, a body-contacting surface or "body surface" 20A and a garment surface 20B. The sanitary napkin 20 is shown in FIG. 1 as viewed from its body surface 20A. The body surface 20A is intended to be worn adjacent to the wearer's body. The garment surface 20B is intended to be placed adjacent to the wearer's undergarments when the sanitary napkin 20 is worn. The sanitary napkin 20 has two centerlines, a principal longitudinal centerline L and a principal transverse centerline T.

FIG. 1 shows that the main body portion 22 of the sanitary napkin 20 comprises the portion of the sanitary napkin without the flaps 24. The main body portion 22 has two spaced apart longitudinal edges 26, two spaced apart transverse or end edges (or "ends") 28, which together form the periphery 30 of the main body portion. The main body portion 22 of the sanitary napkin 20 can be of any thickness, including relatively thick, intermediate thickness, relatively thin, or even very thin (or "ultra thin"). An "ultra-thin" sanitary napkin 20 as described in U.S. Patents 4,950,264 and 5,009,653 issued to Osborn preferably has a caliper of less than about 3 millimeters. The embodiment of the sanitary napkin 20 shown in the drawings is intended to be an example of a sanitary napkin of an intermediate thickness. The main body portion 22 of the sanitary napkin 20 may also be relatively flexible, so that it is comfortable for the wearer. It should be understood that the sanitary napkin shown is merely one embodiment, and that the present invention is not limited to absorbent articles, such as for example those shown in the drawings.

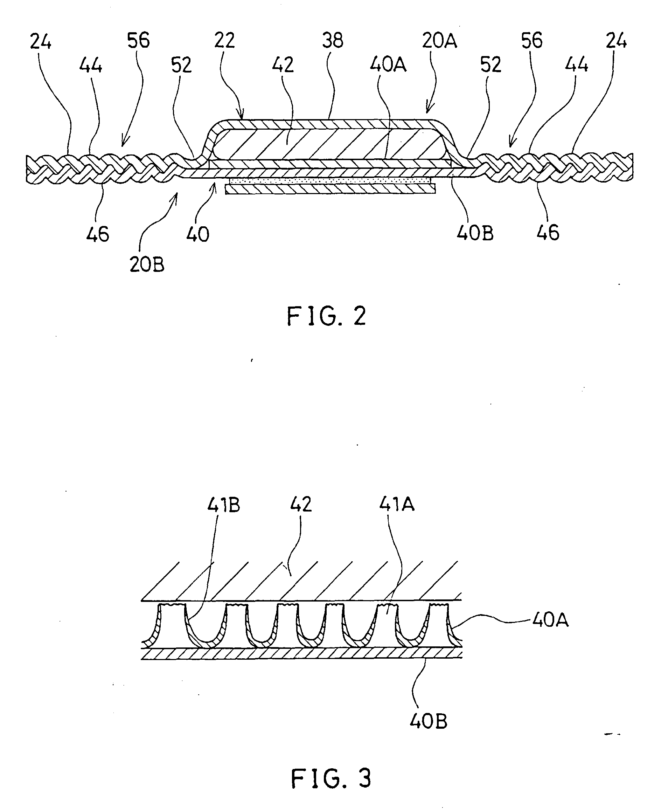

FIG. 2 shows the individual components of the main body portion 22 of the sanitary napkin 20 of the present invention. The main body portion 22 of the sanitary napkin 20 preferably comprises at least three primary components. These include a liquid pervious topsheet 38 typically provided by a liquid permeable substrate of fibrous such as nonwoven or film like structure such as apertured formed films, a liquid impervious backsheet 40 preferably provided by a liquid impermeable, but breathable substrate, and an absorbent core 42 positioned between the topsheet 38 and the backsheet 40. The backsheet 40 comprises two layers; a first layer comprising a gas permeable apertured formed film layer 40A and a second layer comprising a breathable microporous film layer 40B.

The topsheet, the backsheet, and the absorbent core may be assembled in a variety of configurations known in the art (including layered or "sandwich" configurations and wrapped or "tube" configurations). FIGS. 1 and 2 show a preferred embodiment of the sanitary napkin 20 assembled in a sandwich construction in which the topsheet 38 and the breathable microporous film 40B have length and width dimensions generally larger than those of the absorbent core 42. The topsheet 38 and the breathable microporous film 40B extend beyond the edges of the absorbent core 42 to form portions of the periphery 30. The apertured formed film 40A of the backsheet has the approximately same shape as the absorbent core 42 to cover at least the region where the absorbent core 42 lies as shown in FIG. 2. Alternatively, it may have a slightly bigger shape than the absorbent core 42, or may have the same shape as the main body portion 22 of the sanitary napkin 20. In any case, preferably, the apertured formed film 40A does not extend into the flaps 24 as shown in FIG. 2. Alternatively, the apertured formed film 40A may extend into the flaps 24 so that the apertured formed film constitutes a part of the flaps 24.

The topsheet 38 is preferably joined to the body-facing side of the absorbent core 42 and the backsheet 40 (i.e., the apertured formed film 40A) is preferably joined to the garment-facing side of the absorbent core 42. The topsheet 38 and the apertured formed film 40A can be joined to the absorbent core 42 in any suitable manner known in the art for this purpose, such as by an open pattern of adhesives. The portions of the topsheet 38 and the breathable microporous film 40B that extend beyond the edges of the absorbent core are preferably also joined to each other. The topsheet 38 and the breathable microporous film 40B can be joined in any suitable manner known in the art for this purpose. Preferably, in the embodiment shown, these portions of the topsheet 38 and the breathable microporous film 40B are joined using adhesives over substantially the entire portions that extend beyond the edges of the absorbent core 42, and a crimp seal at the end edges 28 of the main body portion where the topsheet 38 and backsheet 40 are densified by the application of pressure or heat and pressure.

The sanitary napkin 20 shown in FIGS. 1 and 2 also comprises a pair of flaps 24 that are joined to the main body portion 22 along a juncture, such as lines of juncture 52. The flaps 24 extend laterally outward beyond the longitudinal side edges 26 of the main body portion 22 from their proximal edges to their distal edges (or "free ends"). The flaps 24 comprise a flap topsheet 44 and a flap backsheet 46. In the embodiment shown in FIGS. 1 and 2, the flaps 24 are integral with the main body portion 22, that is, the flap topsheet 44 and the flap backsheet 46 comprise integral extensions of the topsheet 38 and the breathable microporous film 40B, respectively. In the preferred embodiment, the apertured formed film 40A does not extend into the flaps 24.

The extensions of the topsheet 38 and the breathable microporous film 40B of the flaps 24 (i.e., the flap topsheet 44 and the flap backsheet 46) may be joined by any suitable method, such as adhesive attachment, ultrasonic attachment, heat attachment or the like. In the preferred embodiment, the extensions of the topsheet 38 and the breathable microporous film 40B are joined by applying adhesive to substantially all the region of the flaps 24.

The topsheet 38 is preferably compliant, soft feeling, and non-irritating to the wearer's skin. Further, the topsheet 38 is fluid pervious, permitting fluid to readily penetrate through its thickness. A suitable topsheet 38 may be manufactured from a wide range of materials such as woven and nonwoven materials; polymeric materials such as apertured formed thermoplastic films, apertured plastic films, and hydroformed thermoplastic films; porous foams; reticulated thermoplastic films; and thermoplastic scrims. Suitable woven and nonwoven materials can be comprised of natural fibers (e.g., wood or cotton fibers), synthetic fibers (e.g., polymeric fibers such as polyester, polypropylene, or polyethylene fibers); or from a combination of natural and synthetic fibers. A preferred topsheet comprises an apertured formed film. In the embodiment, apertured formed films are preferably used for the topsheet because they are pervious to body exudates and yet non-absorbent and have a reduced tendency to allow fluids to pass back through and rewet the wearer's skin. Thus, the surface of the formed film which is in contact with the body remains dry, thereby reducing body soiling and creating a more comfortable feel for the wearer. Suitable apertured formed films are described in U.S. Patent No. 3,929,135 issued to Thompson, on December 30, 1975; U.S. Patent No. 4,324,246 issued to Mullane et al., on April 13, 1982; U.S. Patent No. 4,342,314 issued to Radel, et al., on August 3, 1982; U.S. Patent No. 4,463,045, issued to Ahr, et al., on July 31, 1984 and U.S. Pat. No. 5,006,394 issued to Baird, on April 9, 1991.

The backsheet 40 is preferably impervious to liquid and pervious to vapor. The primary role of the backsheet 40 is to prevent the extrudes absorbed and contained in the absorbent core 42 from wetting articles that contact the absorbent product such as underpants, pants, pajamas and undergarments. In addition however, the backsheet 40 also permits the transfer of both vapor and air through it and thus allows the circulation of air into and out of the backsheet 40.

In the embodiment shown in Fig. 2, the backsheet 40 comprises two layers; a first layer comprising a gas permeable apertured formed film layer 40A and a second layer comprising a breathable microporous film layer 40B. The first layer 40A is typically located adjacent to the absorbent core 42 and subsequent layers of the backsheet are typically located further away from the absorbent core 42. The backsheet 40 may comprise additional layers. All of the layers of the backsheet 40 can be substantially in intimate and direct contact with one another.

As shown in FIG. 3 which shows an enlarged cross sectional view of the backsheet 40 with a part of the absorbent core 42, the first layer of the apertured formed film 40A comprises a layer having discrete apertures 41A which extend beyond the horizontal plane of the garment facing surface of the layer towards the absorbent core 42 thereby forming protuberances 41B. Each protuberance 41B has an orifice located at its terminating end. Preferably the protuberances 41B have a funnel or conical shape, similar to those described in US 3,929,135. The apertures located within the plane of the layer and the orifices located at the terminating end of protuberances themselves maybe circular or non circular. In any case the cross sectional dimension or area of the orifice at the termination of the protuberance is smaller than the cross sectional dimension or area of the aperture located within the plane of the layer. The first layer 40A of the backsheet 40 may be made of any material known in the art, but is preferably manufactured from commonly available polymeric materials. The first layer 40A may also comprise any type of formed films which may be used for a topsheet as described above.

The second layer 40B of the backsheet 40 may comprise a breathable microporous film composed of a thermoplastic resin and inorganic fillers dispersed in the thermoplastic resin. Suitable thermoplastic polymers include polyolefins such as polyethylenes, including linear low density polyethylene (LLDPE), low density polyethylene (LDPE), ultra low density polyethylene (ULDPE), high density polyethylene (HDPE), or polypropylene and blends thereof with the above and other materials. Examples of other thermoplastic polymers which may also be used include, but are not limited to, polyester, polyurethanes, compostable or biodegradable polymers, thermoplastic elastomers, and metallocene catalyst-based polymers (e.g., INSITE® available from Dow Chemical Company and Exxact® available from Exxon). The inorganic material or filler can comprise talc, silica, calcium carbonate, clay, titanium dioxide, barium sulfate, with the preferred inorganic filler being calcium carbonate. The inorganic filler may be coated with fatty acid esters, fatty acids or their metal salts to improve the dispersion of the filler particles into the thermoplastic polymer and to obtain higher loadings in the polymer. The inorganic filler and the thermoplastic polymer are blended together to form a homogeneous mixture in a suitable mixing extruder, or in a separate preliminary compounding step. The mixture is then cast or blown into a film. The obtained film is stretched at least in one direction to impart breathability on the substantially entire area of the film. The step of stretching a film to impart breathability may be done at a different place prior to manufacturing process of absorbent articles. Alternatively, the step of stretching may be done at the same place, i.e., same manufacturing process, prior to assembling a breathable microporous film with other elements of absorbent articles. In any case, the film is imparted breathability on the substantially entire area of the film before the resulting breathable microporous film is assembled with other elements of absorbent articles.

The absorbent core 42 may be any absorbent means which is generally compressible, conformable, resilient, non-irritating to the wearer's skin and capable of absorbing and containing body exudates. The absorbent core 42 may be manufactured from a wide variety of fluid absorbent materials commonly used in disposable sanitary napkins, and other disposable absorbent articles. Examples of suitable absorbent materials include comminuted wood pulp (which is generally referred to as airfelt), creped cellulose wadding, modified cross-linked cellulose fibers (such as those described in U.S. Patent No. 5,217,445 issued to Young, et al., on June 8, 1993), capillary channel fibers (that is, fibers having intra-fiber capillary channels such as those described in U.S. Patent No. 5,200,248 issued to Thompson, et al., on April 6, 1993), absorbent foams (such as those described in U.S. Patent No. 5,260,345 issued to DesMarais, et al., on November 9, 1993 and U.S. Patent No. 5,268,244 issued to DesMarais, et al., on December 7, 1993), thermally bonded airlay materials (such as those material described in U.S. Patent No. 5,607,414 issued to Richards, et al., on March 4, 1997), hydrogel-forming polymer gelling agents (such as those material described in U.S. Patent No. 4,673,402 issued to Weisman, et al., on June 16, 1987 and U.S. Patent No. 4,935,022 issued to Lash et al., on June 19, 1990), absorbent sponges, synthetic staple fibers, polymeric fibers, peat moss, or any equivalent materials or combinations of materials. Further, the absorbent core 42 may comprise a first portion and a second portion, the first portion comprising the following components: (a) an optional primary fluid distribution layer preferably together with a secondary optional fluid distribution layer; (b) a fluid storage layer; and the second portion comprising (c) an optional fibrous layer underlying the storage layer; and (d) other optional components. Such a structure is disclosed in PCT publication WO 97/24096 published on July 10, 1997 and WO 97/24095 published on July 10, 1997.

Another component which can be included in the absorbent structure according to the invention and preferably is provided close to or as part of the primary or secondary fluid distribution layer are odour control agents. Typically active carbon coated with or in addition to other odour control agents, in particular suitable zeolite or clay materials, are optionally incorporated in the absorbent structure. These components can be incorporated in any desired form but often are included as discrete particles.

The sanitary napkin 20 shown in FIG. 1 preferably has zones of extensibility (or "zone of differential extensibility") 56 for relieving the stresses on the flaps 24 when they are folded around a panty crotch. The zone of extensibility 56 provides a portion of the sanitary napkin 20 which is capable of extending (and are preferably capable of extending a greater amount than surrounding portions of the sanitary napkin 20). Additionally, the sanitary napkin 20 may have a hinge 54 between the main body portion 22 and at least a portion of the flaps 24. The hinge 54 provides a region of the sanitary napkin 20 with increased flexibility to create preferred bending axes for the flaps 24 to bend or fold about. The hinge 54 may be imparted extensibility. These are further described in PCT publication WO 97/12576 published on April 10, 1997.

The zones of extensibility 56 and hinge 54 can, for instance, be formed by a process which has been described as pre-corrugating (or "ring rolling"). The zones of extensibility 56 and hinge 54 are provided by at least partly, mechanically straining a base material. Suitable methods for ring rolling are described in U.S. Patent 4,107,364 issued to Sisson on August 15, l978, U.S. Patent 4,834,741 issued to Sabee on May 30, l989, U.S. Patent 5,143,679 issued to Gerald M. Weber, et al. on September 1, l992, U.S. Patent 5,156,793 issued to Kenneth B. Buell, et al. on October 20, l992, and U.S. Patent 5,167,897 issued to Gerald M. Weber, et al. on December 1, l992. Alternatively, the zones of extensibility 56 and hinge 54 are provided by forming a strainable network in the region along the juncture 52 of the flaps 24 with the main body portion 22 by mechanically straining a base material. This technology is further described in allowed U.S. Patent Application Serial No. 08/203,087 filed in the name of Chappell, et al. on February 28, l994 (PCT Publication No. WO 95/03765, published February 9, l995).

A base material into which extensibility is imparted comprises a single layer of material or laminate of materials, at least one of which is a breathable microporous film. Preferably, in the embodiment, the base material (composite sheet) that has extensibility therein comprises a laminate formed by an extension of the topsheet 38 and the breathable microporous film 40B.

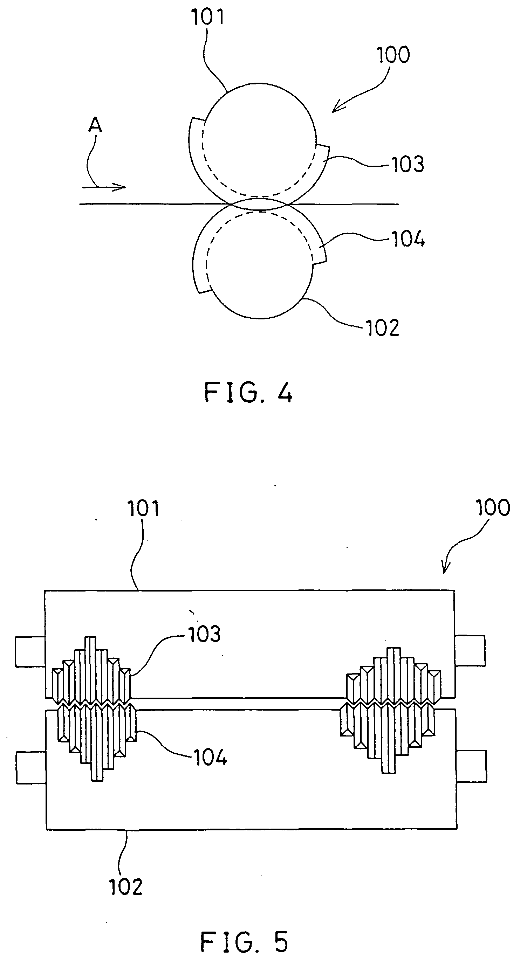

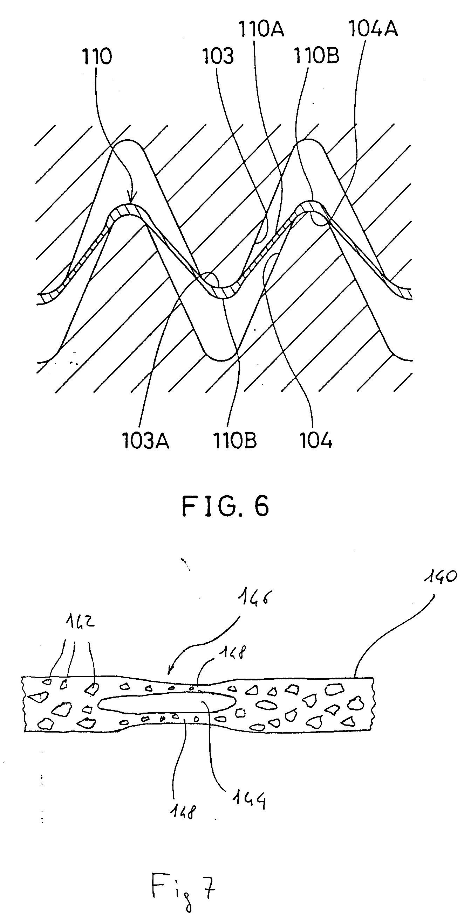

Referring to FIGS. 4 and 5, there is shown a ring rolling unit 100 used to form the zone of extensibility 56 as well as the hinge 54. The ring rolling unit 100 includes intermeshing rolls 101 and 102. The rolls 101, 102 include a plurality of intermeshing teeth 103, 104 respectively on the surfaces of the rolls along the circumferential direction of the rolls 101, 102. In one embodiment, the teeth in this embodiment can have for example a height of 3.175 mm, and are evenly spaced with the centerlines of the teeth spaced apart at 1.9 mm pitches. The entire shape of each toothed regions of the rolls 101 and 102 is generally the same as the entire shape of the zone of extensibility 56 and the hinge 54 of the sanitary napkin 20 shown in FIG. 1. The rolls 101, 102 can be arranged so that the teeth 103 and 104 engage to each other as shown in FIG. 6. The engagement of the teeth 103 and 104 is determined based on desired extensibility. The tooth engagement of 2.11 mm, 2.26 mm and 2.31 mm can be used for example to obtain extensibility of 75 %, 80 % and 85%. The base material 110 positioned between the rolls 101 and 102 is subjected to a strain (applied strain). The term "applied strain" refers to a strain applied to a material by a process in the direction of extensibility to obtain a residual strain for a predetermined extensibility which will be imparted on the material. When the base material 110 is subjected to the applied strain, a portion 110A of the base material 110 between the ridge 103 A of the tooth 103 and the ridge 104A of the tooth 104 is mechanically strained by the applied strain and incrementally and plastically deforms so that a residual strain remains on the base material 110, while portions 110B of the base material 110 on the ridge 103A and 104A are not strained or strained only a little. Since the base material 110 tends to be strained only at the portion 110A between the ridges on the teeth next to each other and the applied strain is not necessarily applied constantly (the applied strain may be sometimes bigger than a desired applied strain or may be smaller than a desired applied strain), the portion 110A of the base material 110 may be strained exceeding a material strain at break of the base material 110. When this occurs, the base material 110 ruptures. The term "material strain at break" refers to a strain at which a material breaks or ruptures.

Extensibility of the zone of extensibility 56 may be from about 35 % to about 100 %. Preferably, extensibility of the zone of extensibility 56 may be from about 65 % to about 90 %. In order to obtain a residual strain for extensibility of, e.g., 75% on the base material, the base material must be strained beyond the residual strain. In one example, the base material comprising an apertured formed film which is marketed as Code No. X-15507 by Tredegar Film Products and a polyethylene film which is marketed as Code No. DH-215 Sofflex Blue 240 by Clopay Plastic Products Company needs to be strained up to 210 % to obtain residual strain for extensibility of 75 % on the base material. In this example, since the polyethylene film is non-microporous film, the base material is capable of being strained up to 210 % without rupturing or creating many visible pin holes. However, the base material comprising a breathable microporous film may not be capable of such high strain without rupturing or many visible pin holes created because a breathable microporous film is weaker against a strain to obtain a residual strain for extensibility than a non-microporous film and easy to rupture. This is because the breathable microporous film undergoes a "second time" strain for obtaining a residual strain, the "first time" strain being applied when stretching the film for imparting breathability. Thus, a breathable microporous film has lower strain at break than a non-microporous film.

Control of the particle size of inorganic fillers of a microporous film is a factor to avoid the base material rupture upon application of a "second time" strain. When the breathable microporous film includes the inorganic fillers with particle size of 10 µm or less, rupturing of the base film during a "second time" strain, e.g. when ring rolling the breathable microporous film in order to obtain a residual strain for a predetermined extensibility, is extremely reduced and visible pin holes that might be possibly created are reduced to the level at which the consumers do not see products as defects. A breathable microporous film including the inorganic fillers with particle size of 10 µm or less works well to obtain e.g. a residual strain for a predetermined extensibility of from about 35 % to about 100% imparted to the material. In the breathable microporous film of the present invention 100% of the particles of inorganic filler have a particle size of 10 µm or less; preferably at least 98% by weight of the particles have a particle size of 5 µm or less, more preferably at least 80% by weight of the particles have a particle size of 2µm or less, and even more preferably at least 60% by weight of the particles have a particle size of 1 µm or less.

The moisture content of the particles of inorganic filler and of the thermoplastic polymer when they are blended to provide the mixture from which the film of the present invention is formed is also a critical parameter in order to obtain a microporous breathable film with an improved extensibility, i.e., which can receive a "second time" strain with a reduced level of rupturing. In breathable microporous films of the prior art the particles of inorganic filler and the thermoplastic polymer, typically in form of pellets, are usually dried before they are homogeneously blended together to provide the mixture which is subsequently extruded or cast in a film. However, it has been observed that the residue moisture contained in the particles of inorganic filler and in the thermoplastic polymer, e.g. in form of pellets, evaporates at the high extrusion temperature and forms cavities in the film that is formed from the mixture. This is illustrated in FIG. 7, where a section of a prior art breathable microporous film 140 is shown. The film is schematically represented after the stretching step ("first time" strain) has been provided to impart breathability, and comprises particles 142 of an inorganic filler. The micropores created by the stretching are not actually represented in the drawing for clarity. The cavity 144 formed within the polymeric matrix of the film 140 by the evaporation of the residue moisture in the mixture of the inorganic filler particles and of the thermoplastic polymer creates a region 146 in the film 140 where the overall thickness of the film, corresponding to the combined thickness of the thin walls 148, is substantially reduced with respect to the average film thickness, owing to the combined effect of the presence of the cavity 144 itself, and of the stretching of the film. The regions 146 constitute weakness areas in the film and therefore, when the film is subsequently stretched, e.g. during a ring rolling step to provide a predetermined extensibility, are more subject to ruptures and to the formation of pin holes.

It has been discovered that a residue moisture content of the inorganic filler particles and of the thermoplastic polymer of not more than 500 ppm, when the particles of the inorganic filler and the thermoplastic polymer are blended together to form the mixture immediately before the casting or extrusion stage to form the film, greatly reduces the occurrence of ruptures and pin holes when the breathable microporous film undergoes a "second time" strain, e.g. in a ring rolling step for providing the film with a predetermined extensibility. Preferably, the moisture content of the inorganic filler particles when they are added to the thermoplastic polymer is less than 300 ppm. By "ppm" is intended a weight percentage corresponding to g/t.

Further, the extensibility of a breathable microporous film according to the present invention can also be improved by controlling the impact resistance of the breathable microporous film, as evaluated according to the Free Falling Dart Test ASTM D1709, Method A. The test measures the resistance of a plastic film under the impact of a dart shaped missile, in terms of the energy that causes the plastic film to fail or rupture. This energy is expressed as the mass in grams of the dart free falling form a specified height which results in a 50% failure of specimens tested. The higher the Dart Resistance value, the higher the extensibility of the breathable microporous film, and therefore the higher the strain that can be applied to the material, e.g. in a ring rolling process in order to impart a predetermined extensibility to the material, with reduced occurrence of ruptures and pin holes.

The Dart Resistance of the breathable microporous film of the present invention is influenced by the particle size of the inorganic filler comprised in the mixture, and by the moisture content of the particles of inorganic filler and of the thermoplastic polymer when they are blended to form the mixture immediately before the formation of the breathable microporous film. However, the Dart Resistance also depends on the nature of the thermoplastic polymer comprised in the breathable microporous film. The Dart Resistance of a polymeric film, e.g. of a breathable microporous film such as that according to the present invention, is inversely proportional to the crystallinity of the thermoplastic polymer, therefore it can be also preferably adjusted by selecting a thermoplastic polymer having a semi amorphous structure. Particularly preferred thermoplastic polymers that can be comprised in the mixture from which the breathable microporous film is formed are polyolefins such as linear low density polyethylene (LLDPE), low density polyethylene (LDPE), and more preferably ultra low or very low density polyethylene (UL/VLDPE).

It has been discovered that a breathable microporous film according to the present invention must have a Dart Resistance value, measured according to the above mentioned test method, of at least 160 g, preferably of at least 180 g.

The microporous breathable film of the present invention can be provided with breathability by stretching in at least one direction (first time strain). In a preferred embodiment of the present invention the microporous breathable film is also imparted a predetermined extensibility in a certain direction in at least designated portions by imparting to the film a mechanical straining in that directions (second time strain), for example by means of a ring rolling process, as mentioned above. It is preferred that the directions of the first time strain and of the second time strain do not coincide. For example, in the embodiment of the present invention illustrated in FIG. 1, where a microporous film is incorporated in a sanitary napkin and has to be provided with a predetermined extensibility in cross direction in the zones 56, e.g. by ring rolling, a microporous film according to the present invention, also being provided with breathability by means of machine direction stretching can be advantageously selected.

The present invention has been so far disclosed in the context of a disposable absorbent article, namely a sanitary napkin, having a backsheet comprising the microporous breathable film of the present invention. However, the microporous breathable film of the present invention, e.g. comprised in a composite layered breathable structure also comprising an apertured formed film, can also be incorporated in different types of products, for example absorbent wound dressings, or in packaging, where a breathable structure which is liquid impervious and water vapour permeable, and moreover has an increased extensibility, can be advantageously used.

A microporous film currently available from Mitsui Petrochemical under the trade name of Espoir PG-01 has been compared to a microporous film according to the present invention. Both are formed by extrusion from a mixture of linear low density polyethylene (LLDPE) as the thermoplastic polymer, and 50% by weight of particles of CaCO₃ as the inorganic filler.

The relevant features are as follows:

| | Espoir PG-01 | Invention |
|---|---|---|
| CaCO₃ particle size | 100% below 25 µm | 100% below 10 µm |
| | 95% below 10 µm | >98% below 5 µm |
| | 84% below 5 µm | >82% below 2 µm |
| | 30% below 1 µm | >60% below 1 µm |
| Moisture content | > 500 ppm | <500 ppm |
| Dart Resistance | 105 g | 180 g |

The two microporous breathable films have been incorporated in a dual layer breathable backsheet of a sanitary napkin as that illustrated in FIGS. 1 and 2 and described above, and subjected to ring rolling in selected zones as shown in the drawings.

The product incorporating the commercial microporous breathable film Espoir PG-01 has shown an incidence of pin holes in the microporous film that can be quantifiable as 1,100 ÷ 27,100 products with at least one pin hole readily visible to the naked eye out of every 1,000,000 of pieces, while the product incorporating the microporous breathable film of the present invention has shown no products with pin holes readily visible to the naked eye.

The expression "readily visible to the naked eye", as used herein to identify pin holes in a microporous breathable film, indicates that said pin holes are readily visible to the naked eye when the perpendicular distance between the viewer's eye and the plane of the film is about 30 cm.

## Claims

1. A breathable microporous film formed from a mixture of a thermoplastic polymer and particles of an inorganic filler, said film being rendered microporous by stretching it at least in one direction,
said breathable microporous film being characterized in that:
said particles of inorganic filler have a particle size of 10 µm or less,
said particles of inorganic filler and said thermoplastic polymer have a moisture content of less than 500 ppm when they are blended to provide said mixture,
said breathable microporous film has a Dart Resistance, as measured according to the Free Falling Dart Test referred to herein, of at least 160 g.

2. A breathable microporous film according to claim 1, characterized in that said particles of inorganic filler and said thermoplastic polymer have a moisture content of less than 300 ppm when they are blended to provide said mixture.

3. A breathable microporous film according to any preceding claim, characterized in that at least 98% by weight of said particles of inorganic filler have a particle size of 5 µm or less.

4. A breathable microporous film according to claim 3, characterized in that at least 80% by weight of said particles of inorganic filler have a particle size of 2 µm or less.

5. A breathable microporous film according to claim 4, characterized in that at least 60% by weight of said particles of inorganic filler have a particle size of 1 µm or less.

6. A breathable microporous film according to any preceding claim, characterized in that said film has a Dart Resistance of at least 180 g.

7. A breathable microporous film according to any preceding claim, characterized in that said thermoplastic polymer comprises a polyolefin resin with a semi amorphous structure such as linear low density polyethylene (LLDPE), preferably low density polyethylene (LDPE), more preferably ultra low/very low density polyethylene (UL/VLDPE).

8. A composite layered breathable structure comprising the breathable microporous film of claim 1 and an apertured formed film.

9. A disposable absorbent article comprising a breathable backsheet, said breathable backsheet comprising the composite layered breathable structure of claim 8.

10. A disposable absorbent article comprising the microporous breathable film of claim 1, wherein at least part of said film is imparted a predetermined extensibility by means of ring rolling by straining at least a part of said microporous breathable film.
